# EUROPEAN PATENT APPLICATION

(11) **EP 1 686 138 A1**
(43) Date of publication of application: **02.08.2006**
(21) Application number: 04799427.2
(22) Date of filing: 01.11.2004
(51) Int. Cl.: C08B 1/00, C07G 1/00, C07H 1/08, C13K 1/02

(54) **METHOD OF SEPARATING AND RECOVERING ACID/SUGAR SOLUTION AND LIGNOPHENOL DERIVATIVE FROM LIGNOCELLULOSIC SUBSTANCE**

(30) Priority: 31.10.2003 JP 2003371705
(71) Applicant: EBARA CORPORATION, Ohta-ku, Tokyo 144-8510 (JP)
(72) Inventor: HAYASHI, Hideaki; c/o Ebara Corporation, Ohta-ku, Tokyo 1448510 (JP); KAMIYA, Ichiro; c/o Ebara Corporation, Ohta-ku, Tokyo 1448510 (JP); KONDO, Kazuhiro; c/o Ebara Corporation, Ohta-ku, Tokyo 1448510 (JP)
(74) Representative: Wagner, Karl H.
(86) International application number: PCT/JP2004/016221
(87) International publication number: WO 2005/042585

(57) **Abstract**

It is an object to provide a method in which a lignocellulosic material is treated with a phenol derivative and acid, whereby a lignophenol derivative can be recovered, and moreover sugar from an acid/sugar solution obtained at the same time can be recovered and used easily and efficiently.
One form of the present invention relates to a method of separating and recovering an acid/sugar solution and a lignophenol derivative, comprising putting a reaction mixture of a lignocellulosic material, a phenol derivative and an acid into an amount of water 0.5 to 6 times the amount of the mixture as a volume ratio, and leaving the mixture to stand or maintaining the mixture in a weakly agitated state, so as to agglomerate a lignophenol derivative produced as a solid phase, and then carrying out solid-liquid separation, so as to separate and recover the lignophenol derivative as the solid phase and an acid/sugar mixture as a liquid phase.

## Description

### TECHNICAL FIELD

The present invention relates to a method and apparatus for efficiently separating and recovering a lignophenol derivative and an acid/sugar solution from a lignocellulosic material. The acid/sugar solution obtained through the present invention can have the sugar recovered therefrom, and thus be used as, for example, a raw material for plant-derived plastic manufacture using, for example, lactic acid fermentation, or can be subjected to carbonization treatment, and thus used as a carbonaceous material for electrodes or the like.

### BACKGROUND ART

The use of fossil resources such as petroleum has become indispensable in modern society, but regeneration of fossil resources is impossible, and so it is feared that these resources will be exhausted in the near future. Interest in biomass resources as one type of resources for replacing fossil resources is thus increasing. Of biomass resources, ligneous biomass resources are receiving attention due to being enormously abundant on Earth, production being possible in a short time period, and sustained supply being possible through appropriate maintenance. Moreover, such ligneous biomass resources are also receiving more and more attention due to decomposing in the natural world after use as resources so as to be regenerated as new biomass resources. To use such a ligneous biomass resource (lignocellulosic material) effectively, it is necessary to first separate the lignocellulosic material into the constituent components thereof, i.e. lignin, and cellulose and hemicellulose. As a technique for doing this, a method has been proposed in which a phenol derivative is impregnated into the lignocellulosic material, and then an acid is added, and the lignocellulosic material is separated into a lignophenol derivative and an acid/sugar solution (Japanese Patent Application Laid-open No. 2-233701; "Synthesis of Functional Lignophenol Derivative using a Natural Lignin Phenol Derivative-Concentrated Acid Two-Phase System Treatment Method", Funaoka et al., Journal of Thermosetting Plastics, Japan, Vol. 15, No. 2 (1994), p. 77-87 (in Japanese); "Derivation of Phenolic Lignin Material using a Phase Separation Reaction System and Functions of the Material", Funaoka et al., Journal of Thermosetting Plastics, Japan, Vol. 16, No. 3 (1995), p. 151-165 (in Japanese)). According to the proposed method, a phenol derivative such as cresol is impregnated into a lignocellulosic material such as wood powder and solvation is carried out (i.e. the cresol is impregnated into the wood powder to produce a state in which the cresol is fixed close to the lignin in the wood powder), and then an acid is added, whereby a cellulose component is solubilized and thus dissolves in an aqueous phase. A lignin component, on the other hand, reacts with the acid, whereby the molecular weight of the lignin is reduced, and a lignophenol derivative in which the phenol derivative is introduced into benzylic positions of the basic structural units is produced. Next, the reaction system (here, this refers to the whole of the reaction liquid after the addition of the acid) is put into a large excess of water, for example an amount of water at least 10 times the amount of the lignocellulosic material, and agitation is carried out to disperse the solid matter, whereby the acid is rapidly diluted so as to stop the reaction with the acid instantly, and then centrifugal separation treatment is carried out, whereby the solid-phase lignophenol derivative is separated off and thus recovered, and moreover a liquid-phase acid/sugar liquid mixture is obtained.

### DISCLOSURE OF THE INVENTION

### PROBLEMS TO BE SOLVED BY THE INVENTION

However, in the above method, after the acid treatment, the reaction system (here this refers to the whole of the reaction liquid after the addition of the acid) is diluted with a large excess of water, for example an amount of water at least 10 times the amount of the lignocellulosic material, and hence the sugar concentration in the acid/sugar solution obtained as the liquid phase is too low, and thus it has been difficult in practice to separate out, recover, and use the sugar. This is because the principal object of the above method is to separate and recover the lignophenol derivative from the lignocellulosic material so that the lignophenol derivative can be used, and there has been little focus on recovering and using the sugar. Accordingly, in the step of diluting the reaction system with water after the acid treatment, the principal object is to reliably stop the reaction with the acid, and hence a technique has been adopted in which the reaction system is put into a large excess of water, and agitation is carried out strongly, thus dispersing the solid matter in the water, and diluting the acid rapidly so as to reliably stop the reaction with the acid. The recovered liquid phase thus contains a large excess of water, and hence the sugar concentration is relatively low, and thus subsequently separating out the sugar has not been practicable.

It is an object of the present invention to solve the above problem, and provide a method in which a lignocellulosic material is treated with a phenol derivative and acid, whereby a lignophenol derivative can be recovered, and moreover sugar from an acid/sugar solution obtained at the same time can be recovered and used easily and efficiently.

### MEANS FOR SOLVING THE PROBLEMS

As means for attaining the above object, in one form of the present invention, there is provided a method of separating and recovering an acid/sugar solution and a lignophenol derivative, comprising putting a reaction mixture of a lignocellulosic material, a phenol derivative and an acid into an amount of water 0.5 to 6 times the amount of the mixture as a volume ratio, and leaving to stand or maintaining a weakly agitated state, so as to agglomerate a lignophenol derivative produced as a solid phase, and then carrying out solid-liquid separation, so as to separate and recover the solid-phase lignophenol derivative and a liquid-phase acid/sugar solution.

### BRIEF DESCRIPTION OF THE DRAWINGS

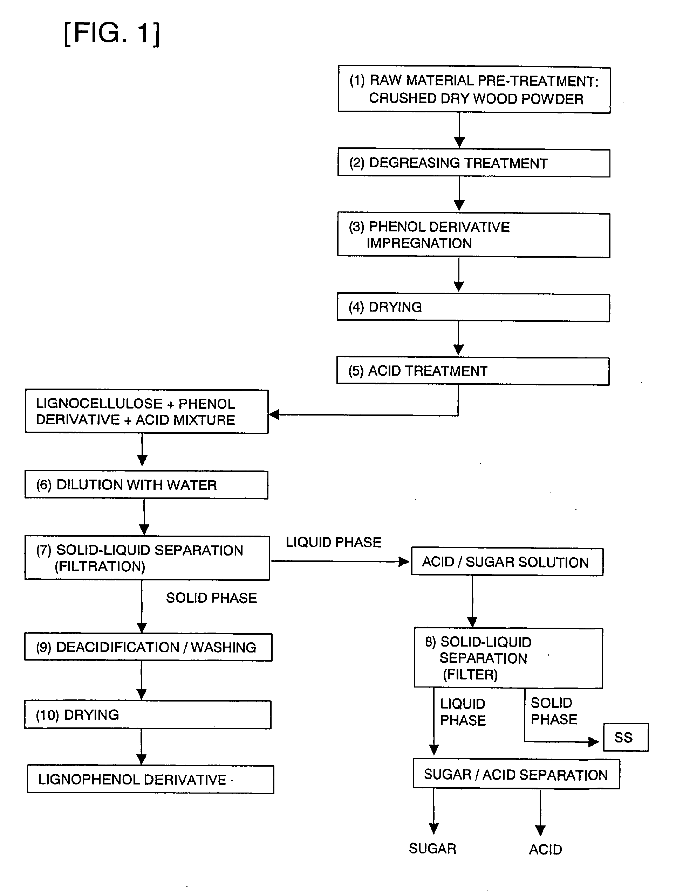

FIG. 1 is a flowchart schematically showing overall a process for manufacturing an acid/sugar solution and a lignophenol derivative from a lignocellulosic material using the present invention;

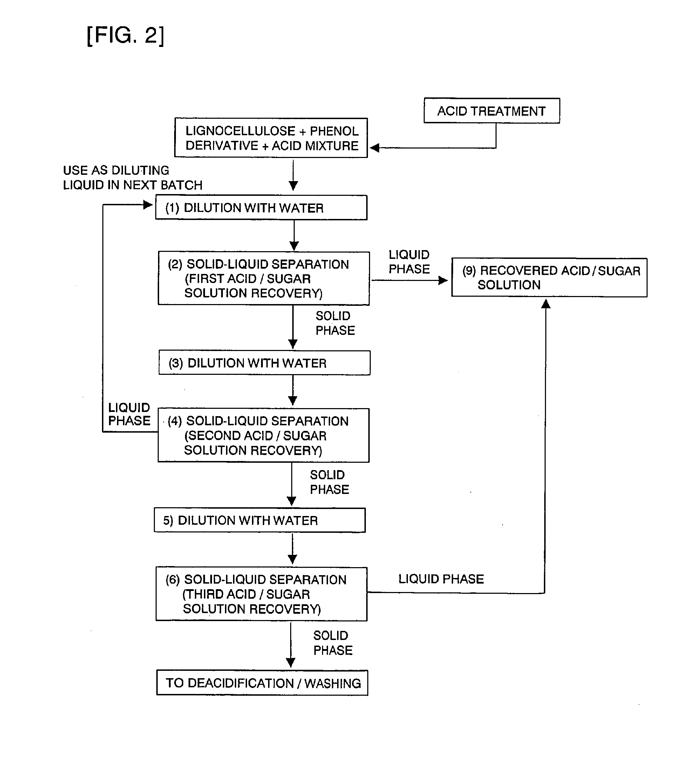

FIG. 2 is a flowchart of the process for manufacturing an acid/sugar solution and a lignophenol derivative from a lignocellulosic material using the present invention, in which to improve the acid/sugar solution recovery rate, dispersion in water and solid-liquid separation are repeated three times, and the acid/sugar solution recovered through the second solid-liquid separation is reused as the first diluting liquid next time; and

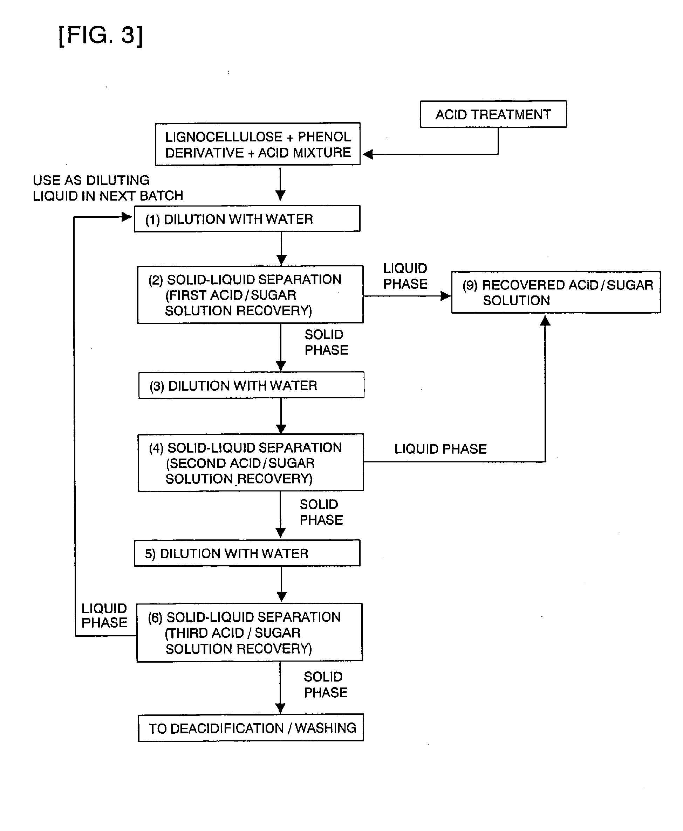

FIG. 3 is a flowchart of the process for manufacturing an acid/sugar solution and a lignophenol derivative from a lignocellulosic material using the present invention, in which to improve the acid/sugar solution recovery rate, dispersion in water and solid-liquid separation are repeated three times, and the acid/sugar solution recovered through the third solid-liquid separation is reused as the first diluting liquid next time.

### MODE FOR CARRYING OUT THE INVENTION

Following is a description of a process for treating a lignocellulosic material so as to separate and recover a lignophenol derivative and an acid/sugar solution according to the present invention. In the following, a description is given of the constitution of the present invention, and also the steps overall in a treatment process that uses the technical idea of the present invention and various representative forms of this treatment process. Accordingly, the technical scope of the present invention is stipulated by the claims, and is not limited by the following description.

FIG. 1 is a flowchart schematically showing overall the process for separating an acid/sugar solution and a lignophenol derivative from a lignocellulosic material using the present invention. In the present invention, a "reaction mixture of the lignocellulosic material, a phenol derivative and an acid" can be prepared using, for example, a method publicly known in the technical field concerned. For example, a lignocellulosic material such as wood or a herbaceous material is first subjected to pre-treatment such as crushing and drying, and degreasing treatment is also carried out as required. Next, the phenol derivative is added to and thus impregnated into the lignocellulosic material. Residual organic solvent is then dried off, and then the acid is added and agitation is carried out, whereby cell membranes in the lignocellulosic material are swollen and destroyed by the acid (acid treatment). As a result, the lignocellulosic material is decomposed into its component elements, i.e. cellulose, hemicellulose, and lignin. The decomposed lignin is reactively bonded to the phenol derivative that has already been added and thus impregnated in, and thus becomes hydrophobic solid matter containing the lignophenol derivative. On the other hand, the molecular weight of the cellulose and hemicellulose is reduced by the acid, whereby solubilization of the cellulose and hemicellulose proceeds. In the present invention, the reaction liquid obtained through the above process is referred to as the "reaction mixture of the lignocellulosic material, the phenol derivative and the acid".

In one form of the present invention, the reaction mixture thus obtained is put into an amount of water not more than 6 times the amount of the mixture as a volume ratio, and the mixture is left to stand or maintained in a weakly agitated state, whereby the lignophenol derivative, which is produced as a solid phase, is agglomerated, and then solid-liquid separation is carried out, thus separating and recovering the solid-phase lignophenol derivative and a liquid-phase acid/sugar solution.

In hitherto trials of techniques in which a phenol derivative and an acid are made to act on a lignocellulosic material and then the lignophenol derivative produced is separated out and recovered, it was very difficult to separate the reaction mixture of the lignocellulosic material, the phenol derivative and the acid into solid matter containing the lignophenol derivative, and an acid/sugar liquid mixture. This is because the reaction caused by the acid proceeds with time, and hence it is necessary to carry out the separation treatment within a prescribed time, and yet the reaction mixture has a high viscosity, and hence separation by leaving to stand is difficult; moreover, due to problems of the fineness of the particles of solid matter, the viscosity of the reaction mixture, and impurities, a filtration method has been similarly difficult to implement, and furthermore centrifugal liquid removal used in combination with a filter cloth has also been difficult to implement. Consequently, a lignocellulosic material treatment process has conventionally been carried out by adding water in a large excess, for example at least 10 times the amount of the lignocellulosic material, to the reaction mixture of the lignocellulosic material, the phenol derivative and the acid, and strongly agitating, so as to dilute the acid rapidly to stop the reaction due to the acid instantly, and moreover disperse solid matter containing the produced lignophenol derivative in the water, and then carrying out solid-liquid separation so as to recover the solid matter. However, from the viewpoint of using the acid/sugar liquid mixture recovered as the liquid phase, this mixture has been too dilute, and hence further treatment of the acid/sugar solution so as to separate and recover the acid and the sugar has been difficult.

Through the research of the present inventors, it has become clear that the main problem causing it to be difficult to carry out solid-liquid separation to recover the acid/sugar solution from the acid-treated reaction mixture is the viscosity of the liquid. Furthermore, the present inventors have found that agglomeration of the solid matter produced can be promoted by putting the mixture into a suitable amount of water and then leaving to stand or maintaining a weakly agitated state. Based on these findings, the present inventors have discovered that by diluting the acid-treated reaction mixture to a suitable extent, the viscosity of the mixture is reduced and hence carrying out the solid-liquid separation is made easy, and thus the solid matter and the liquid phase can be separated easily, whereby the present inventors accomplished the present invention. Furthermore, the present inventors have found that by determing the extent of dilution of the mixture to be within an appropriate range, and moreover leaving the diluted mixture to stand or maintaining the diluted mixture in a weakly agitated state, dispersion of the solid matter in the diluting liquid is suppressed and agglomeration of the solid matter is promoted, and then the agglomerated solid matter can be easily removed.

The lignophenol derivative separated and recovered as the solid phase through the solid-liquid separation is further subjected to deacidification/washing or the like so as to remove impurities contained therein. Moreover, the acid/sugar solution separated and recovered as the liquid phase is not excessively dilute, and hence after being purified through a process such as being left to stand or filtration as required, can be treated using a diffusion dialysis method, a simulated moving bed chromatography separation method, an alkanol solvent extraction method, or the like, whereby the acid and the sugar can be easily separated and recovered.

Following is a detailed description of the various steps, with the flow being shown in FIG. 1.

Raw material pre-treatment: crushing and drying (FIG. 1(1))
The lignocellulosic material, for example thinnings, wood residue of forestry land, sawmill waste, mill ends, herbaceous plants, rice husk, rice straw or the like is crushed. As ligneous raw material, cryptomeria or the like that is wood residue of forestry land or sawmill waste or the like can be suitably used. As herbaceous raw material, the crushed core of kenaf, which has attracted attention recently, or the like can be suitably used. After the crushing, sifting to a particle size of not more than 2 mm is preferably carried out, since this results in an effect of increasing the effectiveness of the subsequent impregnation with the phenol derivative and improving the reactivity. Moreover, it is preferable to carry out drying to a water content of approximately 15 to 20%, since then there is little sticking together of particles to form lumps during the sifting, and hence the yield of the raw material powder can be improved.

Degreasing treatment (FIG. 1(2))
Depending on the type of the lignocellulosic material, the lignocellulosic material may contain a large amount of resinous content or the like. It is preferable to remove the resinous content from the lignocellulosic material (i.e. carry out degreasing) before adding the phenol derivative, so that the resinous content will not inhibit the subsequent reaction process. As the degreasing method, the degreasing can be carried out, for example, by putting the lignocellulosic material and an organic solvent into an agitating tank, and thoroughly mixing and agitating. By carrying out such degreasing with an organic solvent, an effect of removing moisture from the lignocellulosic material is also obtained. Examples of organic solvents that can be used with this objective include acetone and hexane. The amount used of the organic solvent is preferably 1 to 10 times the amount of the lignocellulosic material. "X times the amount" stipulated here means X liters of the organic solvent per 1 kg of the wood powder as a lignocellulosic material, for example "10 times the amount" means that 10 L of the organic solvent is added per 1 kg of the wood powder. Moreover, it is preferable to carry out the degreasing thoroughly by agitating for 1 to 12 hours after the organic solvent has been added. The degreasing treatment is not an essential step, and need not be carried out, for example, in the case that there is not much resinous content in the lignocellulosic material being processed. Note that in the case that the organic solvent used in the present degreasing step is different to an organic solvent used in the following phenol derivative impregnation step, it is preferable to dry the lignocellulosic material so as to remove the organic solvent used in the degreasing before carrying out the following phenol derivative impregnation. However, in the case that the same organic solvent is used in both steps, this drying/removal step may be omitted.

Phenol derivative impregnation (FIG. 1(3))
Next, a solution of the phenol derivative in an organic solvent is mixed with the lignocellulosic material and the mixture is thoroughly agitated, whereby the phenol derivative is impregnated into the lignocellulosic material. Phenol derivatives that can be used with this objective include p-cresol, m-cresol, o-cresol, and mixtures thereof, and also phenol. In this impregnation step, it is desirable to disperse the phenol derivative and impregnate the phenol derivative into the lignocellulosic material thoroughly, and to achieve this it is preferable to make the phenol derivative contact the lignocellulosic material in a state in which the phenol derivative has been mixed and dissolved in an organic solvent and thus thoroughly dispersed through the solvent. Moreover, to efficiently impregnate the phenol derivative into the lignocellulosic material, the solution of the phenol derivative in the organic solvent is preferably added in a proportion of 8 to 12 L per 1 kg of the lignocellulosic material after the degreasing treatment (here, this will be referred to as 8 to 12 times the amount of the lignocellulosic material), preferably approximately 10 times the amount of the lignocellulosic material, so that the impregnation step is carried out in a state in which the lignocellulosic material is thoroughly immersed in the phenol derivative solution. Moreover, the lignocellulosic material and the solution are preferably agitated for 1 to 24 hours at room temperature, for example 10 to 50°C, so that the impregnation proceeds sufficiently, with it being more preferable to maintain a temperature of approximately 30°C during the agitation. Examples of organic solvents that can be used for mixing with and dissolving the phenol derivative include acetone and hexane; in the case of carrying out the degreasing step described above, the same organic solvent as that used in the degreasing step can be used. Examples of apparatuses that can be used for mixing and agitating the lignocellulosic material and the phenol derivative in the organic solvent include a conical ribbon mixer (RIBOCONE made by Okawara Mfg. Co., Ltd.). In the present step, the mixing can be carried out by adding the solution of the phenol derivative in the organic solvent into a mixing tank into which the lignocellulosic material has been put; in this case, it is preferable to reduce the pressure in the mixing tank into which the lignocellulosic material has been put before adding the phenol derivative, since then the penetrability of the phenol derivative into the gaps between the lignocellulosic material particles can be increased, and hence the penetrability of the phenol derivative into the lignocellulosic material cell walls can be increased. Furthermore, as the method of impregnating the phenol derivative into the lignocellulosic material, a pressurized injection method used, for example, for injecting preservatives into wood can be used. This is method in which the pressure in an injection tank into which the lignocellulosic material has been put is reduced, and then the phenol derivative is injected in under pressure. According to this method, the phenol derivative can be made to penetrate as far as the cell membranes of the lignocellulosic material. Note that "impregnation of the phenol derivative into the lignocellulosic material" in the present step does not necessarily mean that the phenol derivative is made to penetrate into the particles of the lignocellulosic material, but rather substantially the same effect can be obtained even if the phenol derivative is merely dispersed and attached very uniformly to the surfaces of the lignocellulosic material particles. This form is thus also included under "impregnation" in the present specification.

Moreover, the present inventors have discovered that in the step of impregnating the phenol derivative into the lignocellulosic material, instead of the method described above in which a phenol derivative solution is added in an amount approximately 10 times the amount of the lignocellulosic material so that the impregnation is carried out in a state in which the lignocellulosic material is thoroughly immersed in the solution, the phenol derivative can be dispersed and attached very uniformly to the surfaces of the lignocellulosic material particles and hence the desired effect can be obtained also through a method in which the phenol derivative solution is added to the lignocellulosic material in a small amount of approximately 1 to 5 times the amount of the lignocellulosic material while agitating the lignocellulosic material. The present invention also relates to such a method. That is, another form of the present invention relates to a method of impregnating the phenol derivative into the lignocellulosic material, in which a phenol derivative solution is added in an amount of 1 to 5 times, preferably approximately 1 times, relative to 1 kg of the crushed lignocellulosic material while agitating the lignocellulosic material. In this case, the amount added of the phenol derivative solution per 1 kg of the lignocellulosic material is more preferably 1 to 4 times, yet more preferably 1 to 2 times.

In this case, the impregnation of the phenol derivative into the lignocellulosic material is preferably carried out by spraying the phenol derivative solution onto the crushed lignocellulosic material while agitating the lignocellulosic material in an agitating apparatus capable of strongly agitating and mixing a powder. The agitating apparatus used in the present invention is an agitating apparatus having plough-shaped shovels and choppers; a stirrer to which these members are attached is rotated, whereby the crushed lignocellulosic material in the tank is subjected to a centrifugal dispersing action and a swirling action to form a state of three-dimensional flow; by spraying the phenol derivative solution onto the crushed lignocellulosic material in this state, a uniformly dispersed state can be realized even with a small amount of liquid. Furthermore, the drying off of the solvent after the impregnation step can also be carried out in the same strongly agitating apparatus, it being possible to greatly reduce the time required for the drying by using the same strongly agitating action as for the impregnation. An example of a strongly agitating apparatus that can be used with this objective is an MFK type Lödige mixer made by the German company Lödige.

By carrying out the impregnation of the phenol derivative into the lignocellulosic material using such a method, the amount used of the solvent can be greatly reduced, and moreover the impregnation can be made more uniform, and furthermore the time taken for the impregnation step can be greatly reduced. For example, with a method in which the impregnation is carried out by thoroughly immersing the lignocellulosic material in approximately 10 times the amount of the phenol derivative solution, it has taken approximately 2 to 3 days up to and including the drying step after the impregnation step, but with the above method, the impregnation and drying steps can be completed in only approximately 1 to 4 hours.

Note that in the case that the impregnation step is carried out by adding the phenol derivative solution to the crushed lignocellulosic material while agitating the lignocellulosic material as described above, in the case that the lignocellulosic material supplied in the impregnation step has had solvent remaining after the degreasing step described earlier removed by drying, or the solvent used in the degreasing step and the solvent used in the impregnation step are the same, the lignocellulosic material used may be obtained by draining off the solvent after the degreasing step (i.e. may having a small amount of the solvent remaining therein).

Furthermore, by carrying out impregnation of the phenol derivative into the lignocellulosic material by adding the phenol derivative solution in an amount of approximately 1 to 5 times relative to the crushed lignocellulosic material while strongly agitating the lignocellulosic material using a Lödige mixer or the like as described above, an effect is also produced whereby the concentration of the phenol derivative solution used in the impregnation can be reduced and hence the amount used of the phenol derivative can be reduced. To prepare the lignophenol derivative effectively, the amount of the phenol derivative impregnated into the lignocellulosic material must be approximately 0.1 to 0.5 kg of the phenol derivative per 1 kg of the lignocellulosic material. With a conventional method, to improve the effect of the impregnation of the phenol derivative into the lignocellulosic material, the impregnation has been carried out by thoroughly immersing the lignocellulosic material in approximately 10 times the amount of the phenol derivative solution. However, with that method, to reduce the heat expense on subsequent drying off of the solvent, a technique of draining off excess phenol derivative solution before the drying is adopted. In this case, the phenol derivative is removed together with the solvent, and hence it is usual to use the phenol derivative in a larger amount than the above, for example 0.3 to 1.5 kg per 1 kg of the wood powder as a lignocellulosic material, when carrying out the impregnation. However, according to the method in which the impregnation of the phenol derivative into the lignocellulosic material is carried out by adding the phenol derivative solution in an amount of approximately 1 to 5 times relative to the crushed lignocellulosic material while strongly agitating the lignocellulosic material using a Lödige mixer or the like as in the present invention, the amount of the phenol derivative used in the phenol derivative impregnation step can be made to be approximately 0.1 to 0.5 kg per 1 kg of the lignocellulosic material. As a result, the amount of the phenol derivative used can be greatly reduced, and moreover the time required for the impregnation and drying steps can be greatly reduced.

Drying (FIG. 1(4))
After the lignocellulosic material and the organic solvent solution having the phenol derivative dissolved therein have been thoroughly agitated so as carry out the impregnation, excess solvent is discharged, and then the pressure is reduced so that residual organic solvent is evaporated off at a low temperature, whereby the phenol derivative-impregnated lignocellulosic material is dried. In the case in particular of using acetone as the organic solvent for dissolving the phenol derivative, the acetone would dissolve the lignophenol derivative produced through the acid treatment in the next stage and thus inhibit the separation of the lignophenol derivative and the acid/sugar solution, and hence it is necessary to thoroughly remove residual acetone in the phenol derivative-impregnated lignocellulosic material before carrying out the acid treatment step.

Acid treatment (FIG. 1(5))
Next, the phenol derivative-impregnated lignocellulosic material is treated with an acid. As the acid used here, it is preferable to use concentrated sulfuric acid of concentration at least 65%. The amount of the acid added is preferably 1 to 10 times the amount, more preferably 3 to 5 times the amount, of the lignocellulosic material. "X times the amount" for the acid here means X liters of the acid per 1 kg of the lignocellulosic raw material before the impregnation of the phenol derivative (i.e. not including the weight of the impregnated phenol derivative), for example "10 times the amount" means that 10 L of the acid is added per 1 kg of the lignocellulosic raw material not including the weight of the impregnated phenol derivative. In the acid treatment step, it is preferable to add the acid after the lignocellulosic material has been put into the reaction tank, since then a reaction time difference can be eliminated, and hence the acid treatment can be carried out uniformly; however, there is no limitation to this, but rather a method in which the phenol derivative-impregnated lignocellulosic material is mixed in after the acid has been put into the reaction tank is also possible. The acid treatment reaction is preferably carried out at a temperature of 20 to 40°C, preferably at least 30°C. Through the research of the present inventors, it has been discovered that by holding the temperature in the reaction tank at 40°C, there is an effect whereby solubilization of cellulose and hemicellulose proceeds, and hence the filtration time in subsequent dilution with water and solid-liquid separation steps can be shortened. Moreover, to prevent denaturation of the lignophenol derivative by the acid, the reaction time for the acid treatment is preferably 10 minutes to 2 hours, more preferably 30 minutes to 1 hour.

Through this acid treatment step, cations at highly reactive sites of the lignin produced through contact with the acid are attacked by the phenol derivative, whereby the phenol derivative is introduced. Moreover benzyl aryl ether linkages are cleaved, whereby the molecular weight of the lignin is reduced. As a result, a lignophenol derivative in which the phenol derivative is introduced into benzylic positions of the basic structural units is produced, and separates away from the liquid phase. Moreover, at the same time, cellulose and hemicellulose in the lignocellulosic material are solubilized by the acid, and thus dissolve in the acidic solution. In the present invention, the mixture thus obtained is referred to as the "reaction mixture of the lignocellulosic material, the phenol derivative and the acid".

Dilution with water (FIG. 1(6))
In one form of the present invention, the reaction mixture of the lignocellulosic material, the phenol derivative and the acid obtained as described above is diluted with an amount of water 0.5 to 6 times the amount of the mixture as a volume ratio, and the mixture is left to stand or maintained in a weakly agitated state, so as to agglomerate the lignophenol derivative produced as a solid phase, and then solid-liquid separation is carried out, so as to separate and recover the lignophenol derivative as the solid phase, and an acid/sugar solution as the liquid phase.

First, the reaction mixture of the lignocellulosic material, the phenol derivative and the acid is put into an amount of water 0.5 to 6 times the amount of the reaction mixture as a volume ratio, or such an amount of water 0.5 to 6 times the amount of the reaction mixture as a volume ratio (the volume ratio relative to the mixture) is put into the reaction mixture, and the mixture is left to stand or maintained in a weakly agitated state. As a result, the acid is diluted, and moreover the viscosity of the mixture is reduced, whereby carrying out solid-liquid separation is made easy. Furthermore, by determining the extent of dilution of the mixture to be within such an appropriate range, and moreover leaving the diluted mixture to stand or maintaining the diluted mixture in a weakly agitated state, agglomeration of the solid matter in the liquid is promoted, and dispersion of the solid matter in the liquid is suppressed, and hence the agglomerated solid matter can be easily removed. However, if the factor of dilution with the water is too high, then the acid/sugar solution recovered will be made too dilute, and hence a subsequent step of recovering the sugar will become very troublesome and difficult to implement. The present inventors carried out a series of experiments, and as a result discovered that to keep down the amount of dispersion of solid matter in the liquid, and to enable refining of the recovered acid/sugar solution to be carried out easily, it is important for the dilution of the reaction mixture to be carried out with an amount of water not more than 6 times the amount of the mixture as a volume ratio. In the present invention, the dilution of the reaction mixture is preferably carried out with an amount of water 0.5 to 6 times, preferably 0.5 to 5 times, more preferably 0.5 to 3 times, the amount of the mixture as a volume ratio. From the viewpoint of the efficiency of separating and recovering the acid and the sugar, it is most preferable to carry out the dilution by adding an amount of water approximately 1 times the amount of the mixture as a volume ratio, i.e. approximately the same volume of water as the mixture. In this dilution with water, to prevent denaturation of the lignophenol derivative due to bonding with Ca ions or Mg ions, it is preferable to use pure water, deionized water, or distilled water.

Moreover, in the present invention, after diluting the reaction mixture with an amount of water 0.5 to 6 times the amount of the mixture as a volume ratio, it is important to leave the mixture to stand or maintain the mixture in a weakly agitated state, so as to dilute the acid, and promote agglomeration of the solid matter. By leaving the reaction mixture to stand or maintaining the reaction mixture in a weakly agitated state after diluting the reaction mixture with an amount of water 0.5 to 6 times the amount of the mixture as a volume ratio in this way, the ability of the acid/sugar solution trapped in the lignophenol derivative produced as a solid phase to be hydrated can be increased, and moreover agglomeration of the lignophenol derivative can be promoted through a hydrophobic effect of the lignophenol derivative. In the conventional method, the reaction mixture was put into a large excess of water and agitation was carried out strongly, and hence the solid-phase lignophenol derivative was widely and finely dispersed through the water, and thus did not agglomerate. This is because in the conventional method, reliably stopping acid reaction with the acid was taken as the principal object. A "weakly agitated state" in the present invention thus means a state of weak agitation to the extent that the lignophenol derivative produced as a solid phase is not finely dispersed in the water, but rather agglomerates to form a flocculent agglomerate. Note that it is particularly preferable to maintain the mixture in a weakly agitated state after the dilution with water, since then acid trapped in the agglomerate of the lignophenol derivative is dispersed through the water and thus diluted. The above operation of diluting with water can be carried out at room temperature.

Solid-liquid separation (FIG. 1(7))
Next, the reaction mixture in which the solid matter has been agglomerated through the dilution with water is separated into an acid/sugar solution as a liquid phase, and solid matter containing the lignophenol derivative as a solid phase. According to the present invention, the reaction mixture is put into a suitable amount of water, or a suitable amount of water is put into the reaction mixture, and the mixture is left to stand or maintained in a weakly agitated state, so as to promote agglomeration of the solid matter; as a result, the viscosity of the liquid drops, and moreover the lignophenol derivative-containing solid matter agglomerates into a state enabling easy separation, and hence solid-liquid separation treatment can be carried out on the reaction mixture easily using a convenient method such as filtration. For example, after the dilution with water, by adding the reaction mixture into a filtration tank equipped with a filter cloth, solid-liquid separation into lignophenol derivative-containing solid matter and an acid/sugar solution can be carried out easily. To carry out the solid-liquid separation effectively, it is preferable to apply pressure or use vacuum filtration.

According to the present invention, through the treatment as described above, the lignophenol derivative and the acid/sugar solution are separated and recovered from the reaction mixture of the lignocellulosic material, the phenol derivative and the acid. The lignophenol derivative recovered as the solid phase is further dispersed in water to completely stop the reaction with the acid, and is then purified through treatment such as deacidification/washing, and can then be used as a plant-derived plastic raw material or the like.

Solid-liquid separation (FIG. 1(8))
The acid/sugar solution recovered as the liquid phase can be treated using a method publicly known in the technical field concerned such as a diffusion dialysis method, a simulated moving bed chromatography separation method, or an alkanol solvent extraction method, whereby the acid and the sugar can be separated. However, with these separation methods, it is important to thoroughly remove impurities from the liquid before the separation; with the diffusion dialysis method in particular, in the case of separating a mixture of pure acid and sugar, a separation performance close to the theoretical value can be obtained, but if there is contamination with impurities, then the desired performance is not obtained, and moreover there is a problem that the membrane separation performance drops in a short time due to fouling of the dialysis membrane. Accordingly, in a preferable form of the present invention, the acid/sugar solution recovered through the dilution with water and solid-liquid separation described above is preferably further subjected to second solid-liquid separation, for example passed through a filter or the like, to remove suspended solids (SS) from the liquid.

In the second solid-liquid separation (FIG. 1(8)) carried out with this objective, because the viscosity of the liquid is not high, and moreover the amount of SS contained in the liquid is low, the SS can be separated out and removed easily using a solid-liquid separation apparatus such as a centrifugal separator, a vacuum filtration apparatus, or a cartridge filter.

Deacidification/washing (FIG. 1(9))
The lignophenol derivative-containing solid matter obtained through the first solid-liquid separation (FIG. 1(7)) is subjected to deacidification/washing to remove impurities such as residual acid/sugar. The solid matter obtained through subjecting the acid-treated reaction mixture to the dilution with water and solid-liquid separation according to the present invention is highly dispersible during the washing, and hence can be dispersed using a general-purpose stirrer, i.e. the solid matter can be dispersed in water without using means such as crushing the solid matter during the dispersion. An apparatus such as a crushing machine or a line mixer may, however, of course be used during the dispersion in water with an objective of improving the effect of the deacidification/washing.

In the present deacidification/washing step, dispersion of the solid matter in water and solid-liquid separation are carried out repeatedly, being carried out repeatedly until the sulfuric acid concentration in the dispersion or the discharged liquid (e.g. the filtrate) reaches a prescribed value, for example the pH reaches approximately 5 to 6. After this prescribed value has been reached, the dispersion of the lignophenol derivative-containing solid matter is subjected to solid-liquid separation using a centrifugal separator or a filtration apparatus, thus recovering the lignophenol derivative-containing solid matter. The amount of water added when carrying out the dispersion in water is preferably made to be 5 to 10 times (weight ratio) the amount of the lignophenol derivative-containing solid matter obtained through the solid-liquid separation. Moreover, as the water used, to prevent denaturation of the lignophenol derivative due to bonding with Ca ions or Mg ions, it is preferable to use pure water, deionized water, or distilled water.

Drying (FIG. 1(10))
After the deacidification/washing has been completed, utilizing the property that the lignophenol derivative will dissolve in acetone, the recovered lignophenol derivative-containing solid matter is mixed with acetone so as to extract only the lignophenol derivative. The extract can be used by being impregnated into a material such as wood, but in this case, if there is residual moisture present when mixing with the acetone, then residual sugar in the lignophenol derivative-containing solid matter will dissolve into the acetone via the moisture, making it difficult to produce a pure lignophenol derivative acetone solution. It is thus preferable to dry the lignophenol derivative-containing solid matter as far as a water content of approximately not more than 5%. To reduce the time required for the drying and thus improve the production efficiency, it is preferable to subject the solid matter first to rough drying to a water content of not more than 50% through drying in a natural air current or drying by blasting with warm air, and then to high-level drying to a water content of not more than 10%. The temperature of the lignophenol derivative during the rough drying is preferably made to be not more than 60°C, and to improve the quality of the lignophenol derivative is more preferably made to be not more than 40°C. In the rough drying, it is preferable to spread the solid matter over a water-absorbent substance, and carry out drying in a natural air current or a warm air blast. The high-level drying can be carried out, for example, by using a vacuum microwave drier, putting the lignophenol derivative-containing solid matter that has been subjected to the rough drying to a water content of not more than 50% into a drying chamber of the drier, reducing the pressure in the drying chamber so as to make the evaporating temperature of water not more than 40°C, and then irradiating the solid matter in the drying chamber with microwaves so as to heat and thus evaporate off the contained moisture. Moreover, by using the above in combination with irradiation of far infrared radiation in the drying chamber, the drying efficiency can be further improved. It is of course also possible to dry lignophenol derivative-solid matter having a water content of approximately 70% obtained through the solid-liquid separation after the deacidification/washing to a water content of approximately not more than 5% using a vacuum microwave drier.

As another form of the present invention, after the reaction mixture of the lignocellulosic material, the phenol derivative and the acid has been diluted with an amount of water 0.5 to 6 times the amount of the mixture as a volume ratio, the lignophenol derivative-containing solid matter recovered in the step of carrying out the solid-liquid separation (FIG. 1(7)) is dispersed in a prescribed amount of water, and then solid-liquid separation is carried out again 1 or 2 times, to further recover acid/sugar solution, which is mixed with the previously recovered acid/sugar solution, whereby the efficiency of recovery of dissolved cellulose/hemicellulose and acid in the reaction mixture of the lignocellulosic material, the phenol derivative and the acid can be increased. The flow for improving the recovery rate of dissolved cellulose/hemicellulose and acid in the reaction mixture of the lignocellulosic material, the phenol derivative and the acid using this method is shown in FIG. 2. According to this method, the lignophenol derivative-containing solid matter obtained through the solid-liquid separation (first acid/sugar solution recovery: FIG. 2(2)) carried out after the reaction mixture of the lignocellulosic material, the phenol derivative and the acid has been diluted with an amount of water 0.5 to 6 times the amount of the mixture as a volume ratio is dispersed in 1 to 2 times the amount of water (FIG. 2(3)), and then solid-liquid separation for second acid/sugar solution recovery is carried out by filtering again or the like (FIG. 2(4)). The lignophenol derivative-containing solid matter obtained here is then dispersed in 1 to 2 times the amount of water (FIG. 2(5)), and then solid-liquid separation for third acid/sugar solution recovery is carried out by filtering yet again or the like (FIG. 2(6)). The acid/sugar solution obtained through the first acid/sugar solution recovery (FIG. 2(2)) is used as the recovered acid/sugar solution in a subsequent separation recovery step (FIG. 2(9)), but this is mixed with the acid/sugar solution obtained through the third acid/sugar solution recovery (FIG. 2(6)), whereby adjustment to an acid concentration and a sugar concentration optimal for an acid/sugar separation recovery apparatus such as a simulated moving bed chromatography separation apparatus is possible. The acid/sugar solution obtained through the second acid/sugar solution recovery (FIG. 2(4)) is used as the diluting water when diluting the acid-treated reaction mixture with water (FIG. 2(1)), whereby the efficiency of recovery of dissolved cellulose/hemicellulose and acid in the reaction mixture of the lignocellulosic material, the phenol derivative and the acid can be increased. At the same time, the amount of dissolved cellulose/hemicellulose and acid discharged out of the system through the washing in subsequent deacidification/washing of the lignophenol derivative-containing solid matter is also reduced.

As another form for improving the acid/sugar solution recovery rate, as shown by the flow in FIG. 3, it is possible for the acid/sugar solution obtained through the first acid/sugar solution recovery (FIG. 3(2)) and the acid/sugar solution obtained through the second acid/sugar solution recovery (FIG. 3(4)) to be mixed together, and used as the starting liquid in the subsequent separation recovery step (FIG. 3(9)), and moreover for the acid/sugar solution obtained through the third acid/sugar solution recovery (FIG. 3(6)) to be used as the diluting water when diluting the acid-treated reaction mixture with water (FIG. 3(1)).

In the flow of each of FIG. 2 and FIG. 3, when using the acid/sugar solution obtained through the second or third acid/sugar solution recovery as the diluting water when diluting the acid-treated reaction mixture with water (FIG. 2(1) or 3(1)), the factor of dilution for each of the first and second acid/sugar solution recoveries is adjusted such that the factor of dilution when diluting the acid-treated reaction mixture with the acid/sugar solution is, for example, 0.5 to 6 times as a volume ratio relative to the mixture.

Moreover, as the water used, to prevent denaturation of the lignophenol derivative due to bonding with Ca ions or Mg ions, it is preferable to use pure water, deionized water, or distilled water.

The lignophenol derivative-containing solid matter obtained through the third acid/sugar solution recovery is subjected to the deacidification/washing and drying steps shown in FIG. 1 from 1(9) onwards, thus recovering the lignophenol derivative.

The present invention further relates to an apparatus for implementing a method as described above. Specifically, another form of the present invention relates to an apparatus for recovering an acid/sugar solution, comprising: an aqueous dilution tank that receives water, and has means for putting a reaction mixture of a lignocellulosic material, a phenol derivative and an acid into the water; a first solid-liquid separation apparatus that receives the diluted reaction mixture, and is for carrying out solid-liquid separation so as to separate off a lignophenol derivative as a solid phase; and a second solid-liquid separation apparatus for further carrying out solid-liquid separation treatment on a liquid phase recovered from the first solid-liquid separation apparatus so as to separate out residual SS as a solid phase.

Furthermore, another form of the present invention relates to an apparatus for recovering an acid/sugar solution, comprising: an aqueous dilution tank that receives water, and has means for putting a reaction mixture of a lignocellulosic material, a phenol derivative and an acid into the water; a first solid-liquid separation apparatus that receives the diluted reaction mixture, and is for carrying out solid-liquid separation so as to separate off a lignophenol derivative as a solid phase; a standing tank for leaving a liquid phase recovered from the first solid-liquid separation apparatus to stand; and a second solid-liquid separation apparatus that receives liquid from the standing tank, and is for further carrying out solid-liquid separation treatment so as to separate out residual SS as a solid phase.

Furthermore, another form of the present invention relates to an apparatus for recovering a lignophenol derivative and an acid/sugar solution, comprising: an acid treatment/aqueous dilution tank that receives a phenol derivative-impregnated lignocellulosic material, and has means for adding an acid to the lignocellulosic material, and means for putting diluting water into a reaction mixture containing the lignocellulosic material on which acid treatment has been carried out through the addition of the acid; a first solid-liquid separation apparatus that receives the diluted reaction mixture, and is for carrying out solid-liquid separation so as to separate off a lignophenol derivative as a solid phase; a second solid-liquid separation apparatus for further carrying out solid-liquid separation treatment on a liquid phase recovered from the first solid-liquid separation apparatus so as to separate out residual SS as a solid phase; an agitating tank that receives the solid matter recovered through the first solid-liquid separation, and is for adding water to the solid matter and agitating; and a third solid-liquid separation apparatus that receives an aqueous slurry recovered from the agitating tank, and is for carrying out solid-liquid separation.

Furthermore, another form of the present invention relates to an apparatus for recovering a lignophenol derivative and an acid/sugar solution, comprising: an acid treatment/aqueous dilution tank that receives a phenol derivative-impregnated lignocellulosic material, and has means for adding an acid to the lignocellulosic material, and means for putting diluting water into a reaction mixture containing the lignocellulosic material on which acid treatment has been carried out through the addition of the acid; a first solid-liquid separation apparatus that receives the diluted reaction mixture, and is for carrying out solid-liquid separation so as to separate off a lignophenol derivative as a solid phase; a second solid-liquid separation apparatus for further carrying out solid-liquid separation treatment on a liquid phase recovered from the first solid-liquid separation apparatus so as to separate out residual SS as a solid phase; a crushing apparatus that receives the solid matter recovered through the first solid-liquid separation, and is for crushing the solid matter; an agitating tank for adding water to the crushed solid matter and agitating; and a third solid-liquid separation apparatus that receives an aqueous slurry recovered from the agitating tank, and is for carrying out solid-liquid separation.

Furthermore, another form of the present invention relates to an apparatus for recovering a lignophenol derivative and an acid/sugar solution, comprising: an acid treatment tank that receives a phenol derivative-impregnated lignocellulosic material, and is for adding an acid to bring about reaction; an aqueous dilution tank that receives a reaction mixture of the lignocellulosic material, the phenol derivative and the acid recovered from the acid treatment tank, and has means for putting in diluting water; a first solid-liquid separation apparatus that receives the diluted reaction mixture, and is for carrying out solid-liquid separation so as to separate off a lignophenol derivative as a solid phase; a second solid-liquid separation apparatus for further carrying out solid-liquid separation treatment on a liquid phase recovered from the first solid-liquid separation apparatus so as to separate out residual SS as a solid phase; an agitating tank that receives the solid matter recovered through the first solid-liquid separation, and is for adding water to the solid matter and agitating; and a third solid-liquid separation apparatus that receives an aqueous slurry recovered from the agitating tank, and is for carrying out solid-liquid separation.

Furthermore, another form of the present invention relates to an apparatus for recovering a lignophenol derivative and an acid/sugar solution, comprising: an acid treatment tank that receives a phenol derivative-impregnated lignocellulosic material, and is for adding an acid to bring about reaction; an aqueous dilution tank that receives a reaction mixture of the lignocellulosic material, the phenol derivative and the acid recovered from the acid treatment tank, and has means for putting in diluting water; a first solid-liquid separation apparatus that receives the diluted reaction mixture, and is for carrying out solid-liquid separation so as to separate off a lignophenol derivative as a solid phase; a second solid-liquid separation apparatus for further carrying out solid-liquid separation treatment on a liquid phase recovered from the first solid-liquid separation apparatus so as to separate out residual SS as a solid phase; a crushing apparatus that receives the solid matter recovered through the first solid-liquid separation, and is for crushing the solid matter; an agitating tank for adding water to the crushed solid matter and agitating; and a third solid-liquid separation apparatus that receives an aqueous slurry recovered from the agitating tank, and is for carrying out solid-liquid separation.

Furthermore, another form of the present invention relates to an apparatus for recovering a lignophenol derivative and an acid/sugar solution as described above, further comprising an agitating tank that receives solid matter recovered from the third solid-liquid separation apparatus, and is for adding water to the solid matter and agitating; and a fourth solid-liquid separation apparatus that receives an aqueous slurry recovered from the agitating tank, and is for carrying out solid-liquid separation.

Furthermore, another form of the present invention relates to an apparatus for recovering a lignophenol derivative and an acid/sugar solution as described above, further comprising a crushing apparatus that receives solid matter recovered from the third solid-liquid separation apparatus, and is for crushing the solid matter; an agitating tank for adding water to the crushed solid matter and agitating; and a fourth solid-liquid separation apparatus that receives an aqueous slurry recovered from the agitating tank, and is for carrying out solid-liquid separation.

Furthermore, another form of the present invention relates to an apparatus for recovering a lignophenol derivative and an acid/sugar solution as described above, further comprising means for supplying a liquid phase recovered from the third solid-liquid separation apparatus into the acid treatment/aqueous dilution tank or the aqueous dilution tank as a diluting liquid.

Furthermore, another form of the present invention relates to an apparatus for recovering a lignophenol derivative and an acid/sugar solution as described above, further comprising means for supplying a liquid phase recovered from the fourth solid-liquid separation apparatus into the acid treatment/aqueous dilution tank or the aqueous dilution tank as a diluting liquid.

Moreover, in the apparatus for recovering a lignophenol derivative and an acid/sugar solution of each of the above forms, the first solid-liquid separation apparatus and the third solid-liquid separation apparatus may be constituted from the same solid-liquid separation apparatus, with an aqueous slurry obtained by adding water to and agitating the solid matter after the solid-liquid separation treatment has been carried out through solid-liquid separation (the solid-liquid separation carried out using the first solid-liquid separation apparatus) being returned into the same solid-liquid separation apparatus and once again being subjected to solid-liquid separation (the solid-liquid separation carried out using the third solid-liquid separation apparatus). Similarly, the first solid-liquid separation apparatus and the fourth solid-liquid separation apparatus, or the third solid-liquid separation apparatus and the fourth solid-liquid separation apparatus, or all of the first, third and fourth solid-liquid separation apparatuses may be constituted from the same solid-liquid separation apparatus.

The acid/sugar solution recovered as the liquid phase through the solid-liquid separation (4) can be subsequently treated using a method publicly known in the technical field concerned such as a diffusion dialysis method, a simulated moving bed chromatography separation method, or an alkanol solvent extraction method, whereby the acid and the sugar can be separated and recovered. The recovered sugar can, for example, be used as a raw material for biodegradable plastic manufacture using, for example, lactic acid fermentation.

### Examples

The present invention will now be described in more detail through the following examples. However, the present invention is not limited to the following description.

### Example 1

A cryptomeria wood powder obtained by crushing cryptomeria chips, then drying, and then sifting to 0.2 to 2 mm was used as a raw material. 1 kg of the cryptomeria wood powder was put into an agitating tank (RIBOCONE), 10 L of acetone was added, and agitation was carried out for 24 hours, thus carrying out first degreasing treatment. The acetone (7L) was then discharged, the same amount of acetone as the discharged amount was re-added, and agitation was carried out for 24 hours, thus carrying out second degreasing treatment. After the second degreasing treatment had been completed, a mixture of 500 g of p-cresol and 6 L of acetone was added, and agitation was carried out thoroughly, thus impregnating the p-cresol into the cryptomeria wood powder. After leaving to stand for 24 hours, the pressure in the tank was reduced, thus thoroughly drying off residual acetone. The above degreasing and p-cresol impregnation were carried out at room temperature (15°C).

1.5 kg of the p-cresol-impregnated cryptomeria wood powder was put into an agitating reaction tank, and 72% sulfuric acid was added in an amount of 5 L, i.e. 5 times the amount relative to the cryptomeria wood powder, thus carrying out acid treatment. The agitating reaction tank and the added sulfuric acid used in the acid treatment were warmed to a temperature of 30°C in advance and held at this temperature. The mixture was agitated thoroughly for 1 hour in the reaction tank so as to cause the reaction to proceed, and then the mixture was put into a vessel containing water in the same amount (6.5 L) as the mixture, and slight agitation was carried out, thus diluting the acid, and agglomerating a produced lignophenol derivative. The mixture was then subjected to screen filtration, whereby solid-liquid separation into the agglomerated lignophenol derivative, and a sulfuric acid/sugar solution could easily be carried out.

The separated lignophenol derivative was re-dispersed by adding water, and washed with water repeatedly, whereby residual sulfuric acid was washed out, and hence the lignophenol derivative was recovered.

Moreover, the separated off sulfuric acid/sugar solution was subjected to membrane separation using a filter, thus removing SS dispersed in the solution.

Example 2
A cryptomeria wood powder obtained by crushing cryptomeria chips, then drying, and then sifting to 0.2 to 2 mm was used as a raw material. 100 kg of the cryptomeria wood powder was put into an agitating tank (RIBOCONE), 80 L of acetone was added, and agitation was carried out for 24 hours, thus carrying out degreasing treatment. After the degreasing, the acetone solution (55 L) was discharged, and then the pressure in the agitating tank was reduced so as to thoroughly dry off residual acetone, whereby 83 kg of degreased cryptomeria wood powder was prepared. 10 kg of the degreased cryptomeria wood powder was put into an agitating tank for impregnation (a Lödige mixer), 20 L of an acetone solution having 3 kg of p-cresol dissolved therein was sprayed and strong agitation was carried out for approximately 30 minutes, and then the pressure in the agitating tank was reduced so as to thoroughly dry off residual acetone, whereby 13 kg of p-cresol-impregnated cryptomeria wood powder was prepared. The above degreasing and p-cresol impregnation were carried out at room temperature (15°C).

13 kg of the p-cresol-impregnated cryptomeria wood powder was put into an agitating reaction tank, and 72% sulfuric acid was added in an amount of 28 L, i.e. 3 times the amount relative to the cryptomeria wood powder, thus carrying out acid treatment. The agitating reaction tank and the added sulfuric acid used in the acid treatment were at room temperature 25°C. Agitation was carried out thoroughly for 1 hour in the reaction tank, which was held at 40°C using a hot water jacket, so as to cause the reaction to proceed, and then 37 L of deionized water (0.9 times the volume of the acid-treated mixture) was put into the agitating reaction tank, and slight agitation was carried out, thus diluting the acid, and agglomerating a produced lignophenol derivative. The mixture was then subjected to vacuum filtration, whereby solid-liquid separation into 20 kg of solid matter containing the agglomerated lignophenol derivative, and 75 kg of a sulfuric acid/sugar solution could easily be carried out (first sulfuric acid/sugar solution recovery).

23 L of deionized water (1.2 times the volume of the solid matter) was put into the solid matter obtained through the solid-liquid separation in the first sulfuric acid/sugar solution recovery and the solid matter was dispersed in the water, and then vacuum filtration was carried out, whereby solid-liquid separation into 18 kg of solid matter containing the lignophenol derivative, and 24 kg of a sulfuric acid/sugar solution could easily be carried out (second sulfuric acid/sugar solution recovery). The sulfuric acid/sugar solution obtained through the second sulfuric acid/sugar solution recovery was used in subsequent batches as the water for diluting the reaction mixture obtained through carrying out the acid treatment on the p-cresol-impregnated cryptomeria wood powder.

24 L of deionized water (1.2 times the volume of the solid matter) was put into the solid matter obtained through the solid-liquid separation in the second sulfuric acid/sugar solution recovery and the solid matter was dispersed in the water, and then vacuum filtration was carried out, whereby solid-liquid separation into 17 kg of solid matter containing the lignophenol derivative, and 24 kg of a sulfuric acid/sugar solution could easily be carried out (third sulfuric acid/sugar solution recovery).

In subsequent batches, by using the sulfuric acid/sugar solution obtained through the second sulfuric acid/sugar solution recovery in the previous batch as the liquid for diluting the acid-treated reaction mixture, the recovery rate relative to the acid-treated raw material for a recovered sulfuric acid/sugar solution obtained by mixing together the sulfuric acid/sugar solutions obtained through the first sulfuric acid/sugar solution recovery and the third sulfuric acid/sugar solution recovery after the acid treatment has been carried out on the p-cresol-impregnated cryptomeria wood powder was increased to 98% from 87% for the case of carrying out the dilution using fresh water. Moreover, the sulfuric acid concentration in the recovered sulfuric acid/sugar solution was 25%, and the sugar concentration therein was 5%, and hence it was possible to obtain optimal concentrations for the case of subsequently separating and recovering the sulfuric acid and the sugar using a simulated moving bed chromatography separation method.

Regarding the sulfuric acid/sugar solution recovered using the vacuum filtration apparatus, a clear liquid having no solid matter therein was obtained.

The lignophenol derivative-containing solid matter obtained through the third sulfuric acid/sugar solution recovery was re-dispersed by adding deionized water thereto in an amount of 40 L, which is approximately 5 times the amount of the solid matter, and vacuum filtration was carried out repeatedly, whereby residual sulfuric acid was washed out, and hence the lignophenol derivative was recovered. The dry weight of the recovered lignophenol derivative was 4.2 kg, and hence the yield obtained was 42% relative to the dry cryptomeria wood powder.

### INDUSTRIAL APPLICABILITY

According to the present invention, in the case of a method of treating a lignocellulosic material with a phenol derivative and an acid so as to separate and recover a lignophenol derivative and an acid/sugar solution, the mixture after the acid treatment is put into a small amount of water approximately 1 times the amount of the mixture as a volume ratio, or such an amount of water approximately 1 times the amount of the mixture is put into the mixture, and the mixture is left to stand or maintained in a weakly agitated state, whereby the acid is diluted, and moreover the produced lignophenol derivative is agglomerated in the water, and thus can be easily separated and recovered by screen filtration or the like. Moreover, because the mixture after the acid treatment is not excessively diluted, the separated acid/sugar solution, after being purified through a process such as being left to stand or filtration as required, can be treated using any of various methods commonly used in the technical field concerned such as a diffusion dialysis method, a simulated moving bed chromatography separation method, or an alkanol solvent extraction method, whereby the acid and the sugar can be easily and conveniently separated and recovered.

## Claims

1. A method of separating and recovering an acid/sugar solution and a lignophenol derivative, comprising putting a reaction mixture of a lignocellulosic material, a phenol derivative and an acid into an amount of water 0.5 to 6 times the amount of the mixture as a volume ratio, and leaving to stand or maintaining a weakly agitated state, so as to agglomerate a lignophenol derivative produced as a solid phase, and then carrying out solid-liquid separation, so as to separate and recover the solid-phase lignophenol derivative and a liquid-phase acid/sugar solution.

2. The method according to claim 1, wherein the reaction mixture of the lignocellulosic material, the phenol derivative and the acid is diluted with an amount of water substantially the same as the amount of the reaction mixture as a volume ratio.

3. The method according to claim 1 or 2, wherein the solid-liquid separation is carried out using a filtration apparatus.

4. The method according to any of claims 1 through 3, wherein the acid/sugar solution recovered as the liquid phase through the solid-liquid separation is further subjected to second solid-liquid separation so as to remove residual SS as a solid phase.

5. The method according to any of claims 1 through 4, wherein lignophenol derivative-containing solid matter recovered as the solid phase through the solid-liquid separation is further subjected to third and fourth dispersion in water and solid-liquid separation, whereby the recovery rate for the acid and the sugar in the reaction mixture of the lignocellulosic material, the phenol derivative and the acid is improved.

6. The method according to claim 5, wherein a liquid phase obtained from the third solid-liquid separation and/or the fourth solid-liquid separation is used as diluting water to be put into the reaction mixture of the lignocellulosic material, the phenol derivative and the acid is put.

7. An apparatus for recovering an acid/sugar solution, comprising: an aqueous dilution tank that receives water, and has means for putting a reaction mixture of a lignocellulosic material, a phenol derivative and an acid into the water; a first solid-liquid separation apparatus that receives the diluted reaction mixture, and is for carrying out solid-liquid separation so as to separate off a lignophenol derivative as a solid phase; and a second solid-liquid separation apparatus for further carrying out solid-liquid separation treatment on a liquid phase recovered from the first solid-liquid separation apparatus so as to separate out residual SS as a solid phase.

8. An apparatus for recovering an acid/sugar solution, comprising: an aqueous dilution tank that receives water, and has means for putting a reaction mixture of a lignocellulosic material, a phenol derivative and an acid into the water; a first solid-liquid separation apparatus that receives the diluted reaction mixture, and is for carrying out solid-liquid separation so as to separate off a lignophenol derivative as a solid phase; a standing tank for leaving a liquid phase recovered from the first solid-liquid separation apparatus to stand; and a second solid-liquid separation apparatus that receives liquid from the standing tank, and is for further carrying out solid-liquid separation treatment so as to separate out residual SS as a solid phase.

9. An apparatus for recovering a lignophenol derivative and an acid/sugar solution, comprising: an acid treatment/aqueous dilution tank that receives a phenol derivative-impregnated lignocellulosic material, and has means for adding an acid to the lignocellulosic material, and means for putting diluting water into a reaction mixture containing the lignocellulosic material on which acid treatment has been carried out through the addition of the acid; a first solid-liquid separation apparatus that receives the diluted reaction mixture, and is for carrying out solid-liquid separation so as to separate off a lignophenol derivative as a solid phase; a second solid-liquid separation apparatus for further carrying out solid-liquid separation treatment on a liquid phase recovered from the first solid-liquid separation apparatus so as to separate out residual SS as a solid phase; an agitating tank that receives the solid matter recovered through the first solid-liquid separation, and is for adding water to the solid matter and agitating; and a third solid-liquid separation apparatus that receives an aqueous slurry recovered from the agitating tank, and is for carrying out solid-liquid separation.

10. An apparatus for recovering a lignophenol derivative and an acid/sugar solution, comprising: an acid treatment/aqueous dilution tank that receives a phenol derivative-impregnated lignocellulosic material, and has means for adding an acid to the lignocellulosic material, and means for putting diluting water into a reaction mixture containing the lignocellulosic material on which acid treatment has been carried out through the addition of the acid; a first solid-liquid separation apparatus that receives the diluted reaction mixture, and is for carrying out solid-liquid separation so as to separate off a lignophenol derivative as a solid phase; a second solid-liquid separation apparatus for further carrying out solid-liquid separation treatment on a liquid phase recovered from the first solid-liquid separation apparatus so as to separate out residual SS as a solid phase; a crushing apparatus that receives the solid matter recovered through the first solid-liquid separation, and is for crushing the solid matter; an agitating tank for adding water to the crushed solid matter and agitating; and a third solid-liquid separation apparatus that receives an aqueous slurry recovered from the agitating tank, and is for carrying out solid-liquid separation.

11. An apparatus for recovering a lignophenol derivative and an acid/sugar solution, comprising: an acid treatment tank that receives a phenol derivative-impregnated lignocellulosic material, and is for adding an acid to bring about reaction; an aqueous dilution tank that receives a reaction mixture of the lignocellulosic material, the phenol derivative and the acid recovered from the acid treatment tank, and has means for putting in diluting water; a first solid-liquid separation apparatus that receives the diluted reaction mixture, and is for carrying out solid-liquid separation so as to separate off a lignophenol derivative as a solid phase; a second solid-liquid separation apparatus for further carrying out solid-liquid separation treatment on a liquid phase recovered from the first solid-liquid separation apparatus so as to separate out residual SS as a solid phase; an agitating tank that receives the solid matter recovered through the first solid-liquid separation, and is for adding water to the solid matter and agitating; and a third solid-liquid separation apparatus that receives an aqueous slurry recovered from the agitating tank, and is for carrying out solid-liquid separation.

12. An apparatus for recovering a lignophenol derivative and an acid/sugar solution, comprising: an acid treatment tank that receives a phenol derivative-impregnated lignocellulosic material, and is for adding an acid to bring about reaction; an aqueous dilution tank that receives a reaction mixture of the lignocellulosic material, the phenol derivative and the acid recovered from the acid treatment tank, and has means for putting in diluting water; a first solid-liquid separation apparatus that receives the diluted reaction mixture, and is for carrying out solid-liquid separation so as to separate off a lignophenol derivative as a solid phase; a second solid-liquid separation apparatus for further carrying out solid-liquid separation treatment on a liquid phase recovered from the first solid-liquid separation apparatus so as to separate out residual SS as a solid phase; a crushing apparatus that receives the solid matter recovered through the first solid-liquid separation, and is for crushing the solid matter; an agitating tank for adding water to the crushed solid matter and agitating; and a third solid-liquid separation apparatus that receives an aqueous slurry recovered from the agitating tank, and is for carrying out solid-liquid separation.

13. The apparatus for recovering a lignophenol derivative and an acid/sugar solution according to claim 11 or 12, wherein the first solid-liquid separation apparatus and the third solid-liquid separation apparatus are constituted from the same apparatus.

14. The apparatus for recovering a lignophenol derivative and an acid/sugar solution according to any of claims 9 through 13, further comprising an agitating tank that receives solid matter recovered from the third solid-liquid separation apparatus, and is for adding water to the solid matter and agitating; and a fourth solid-liquid separation apparatus that receives an aqueous slurry recovered from the agitating tank, and is for carrying out solid-liquid separation.

15. The apparatus for recovering a lignophenol derivative and an acid/sugar solution according to any of claims 9 through 13, further comprising: a crushing apparatus that receives solid matter recovered from the third solid-liquid separation apparatus, and is for crushing the solid matter; an agitating tank for adding water to the crushed solid matter and agitating; and a fourth solid-liquid separation apparatus that receives an aqueous slurry recovered from the agitating tank, and is for carrying out solid-liquid separation.

16. The apparatus for recovering a lignophenol derivative and an acid/sugar solution according to claim 14 or 15, wherein the first solid-liquid separation apparatus and the fourth solid-liquid separation apparatus are constituted from the same apparatus.

17. The apparatus for recovering a lignophenol derivative and an acid/sugar solution according to claim 14 or 15, wherein the third solid-liquid separation apparatus and the fourth solid-liquid separation apparatus are constituted from the same apparatus.

18. The apparatus for recovering a lignophenol derivative and an acid/sugar solution according to any of claims 14 through 17, further comprising means for supplying a liquid phase recovered from the third solid-liquid separation apparatus into the acid treatment/aqueous dilution tank or the aqueous dilution tank as a diluting liquid.

19. The apparatus for recovering a lignophenol derivative and an acid/sugar solution according to any of claims 14 through 17, further comprising means for supplying a liquid phase recovered from the fourth solid-liquid separation apparatus into the acid treatment/aqueous dilution tank or the aqueous dilution tank as a diluting liquid.
